# EUROPEAN PATENT SPECIFICATION

(11) **EP 3 463 339 B1**
(45) Date of publication and mention of the grant of the patent: **06.12.2023**
(21) Application number: 17726968.5
(22) Date of filing: 05.06.2017
(51) Int. Cl.: A61K 31/415, A61K 31/4155, A61K 31/5377, A61K 31/337, A61P 35/00

(54) **USE OF SIGMA RECEPTOR LIGANDS IN CANCER**
VERWENDUNG VON SIGMA-REZEPTOR-LIGANDEN BEI KREBS
UTILISATION DE LIGANDS DE RÉCEPTEUR SIGMA DANS LE CANCER

(30) Priority: 06.06.2016 EP 16382258
(43) Date of publication of application: 10.04.2019
(73) Proprietor: ESTEVE PHARMACEUTICALS, S.A., 08038 Barcelona (ES)
(72) Inventor: ZAMANILLO-CASTANEDO, Daniel, E-08041 Barcelona (ES); PRADOS-SALAZAR, José-Carlos, E-18198 Huetor Vega - Granada (ES); VIDELA-CÉS, Sebastià, E-08017 Barcelona (ES); VELA HERNÁNDEZ, José-Miguel, E-08028 Barcelona (ES); PLATA-SALAMAN, Carlos-Ramón, E-08950 Esplugues de Llobregat - Barcelona (ES); BRUNA-ESCUER, Jordi, E-08011 Barcelona (ES)
(74) Representative: ABG Intellectual Property Law, S.L.
(86) International application number: PCT/EP2017/063614
(87) International publication number: WO 2017/211763

(56) References cited:
- EP-A1- 2 292 236
- EP-A1- 2 388 005
- WO-A1-2010/128309
- WANG B ET AL: "EXPRESSION OF SIGMA 1 RECEPTOR IN HUMAN BREAST CANCER", BREAST CANCER RESEARCH AND TREATMENT, SPRINGER , NY, US, vol. 87, no. 3, 1 January 2004 (2004-01-01), pages 205-214, XP008060394, ISSN: 0167-6806, DOI: 10.1007/S10549-004-6590-0
- AMALIA AZZARITI ET AL: "MC70 potentiates doxorubicin efficacy in colon and breast cancer in vitro treatment", EUROPEAN JOURNAL OF PHARMACOLOGY, ELSEVIER SCIENCE, NL, vol. 670, no. 1, 15 August 2011 (2011-08-15), pages 74-84, XP028312051, ISSN: 0014-2999, DOI: 10.1016/J.EJPHAR.2011.08.025 [retrieved on 2011-09-05]
- MIREILLE HAPPY ET AL: "Sigma 1 Receptor antagonist potentiates the anti-cancer effect of p53 by regulating ER stress, ROS production, Bax levels, and caspase-3 activation", BIOCHEMICAL AND BIOPHYSICAL RESEARCH COMMUNICATIONS, vol. 456, no. 2, 1 January 2015 (2015-01-01), pages 683-688, XP055319218, AMSTERDAM, NL ISSN: 0006-291X, DOI: 10.1016/j.bbrc.2014.12.029
- AREN VAN WAARDE ET AL: "Potential applications for sigma receptor ligands in cancer diagnosis and therapy", BIOCHIMICA ET BIOPHYSICA ACTA (BBA) - BIOMEMBRANES, vol. 1848, no. 10, 1 October 2015 (2015-10-01), pages 2703-2714, XP055318889, AMSTERDAM, NL ISSN: 0005-2736, DOI: 10.1016/j.bbamem.2014.08.022
- Deborah L. Gilmore ET AL: "Review of the Pharmacological and Clinical Profile of Rimcazole", CNS DRUG REVIEWS., vol. 10, no. 1, 1 March 2004 (2004-03-01), pages 1-22, XP055686704, US ISSN: 1080-563X, DOI: 10.1111/j.1527-3458.2004.tb00001.x
- ROMERO L ET AL: "Pharmacological properties of S1RA, a new sigma-1 receptor antagonist that inhibits neuropathic pain and activity-induced spinal sensitization", BRITISH JOURNAL OF PHARMACOLOGY, JOHN WILEY & SONS LTD, GB, vol. 166, no. 8, 1 August 2012 (2012-08-01), pages 2289-2306, XP002720183, ISSN: 1476-5381, DOI: 10.1111/J.1476-5381.2012.01942.X

## Description

### FIELD OF THE INVENTION

The present invention relates to the use of sigma receptor ligands, and more particularly to some pyrazole derivatives, and to the use of pharmaceutical compositions comprising them for increasing the efficacy of a chemotherapeutic agent and/or for reducing the cancer cells proliferation in the treatment of cancer.

### BACKGROUND OF THE INVENTION

Cancer and its associated therapies are some of the biggest health concerns in the world.

Although neoplasia involves many other processes that also present targets for cancer therapy, in almost all instances, deregulated cell proliferation and suppressed cell death together provide the underlying platform for neoplastic progression.

Because deregulated proliferation and inhibition of apoptosis lie at the heart of all tumor development, they present two targets of choice for therapeutic intervention in all cancers.

Chemotherapy, in combination with or as an alternative to surgery, is the method of choice in most cases for controlling or helping patients struck by carcinomas.

Chemotherapy is generally defined as the use of chemical substances to treat cancer, tumors or malign neoplasia and, in the sense of this invention, refers to the use of cytotoxic or cytostatic drugs, called chemotherapeutic drugs. In general, it is a systemic treatment. Chemotherapy in cancer treatment consists of a personalized combination of potent chemotherapy drugs, designed to slow rapid cancer tumor growth, shrink tumors, kill cancer cells, and prevent the spread of cancer. The chemotherapeutic drugs prevent cells from replicating in the typical, out-of-control manner in which cancer cells divide.

Chemotherapy-induced peripheral neuropathy (CIPN) is defined as the presence of signs or symptoms of peripheral nerve dysfunction, whether somatic or autonomic, because of damage to the peripheral or autonomic nervous system caused by chemotherapeutic agents (Zhang; JBI Database Systematic Rev. Implement. Reports; 2014; 12; 484-500). CIPN is the most prevalent neurological complication of cancer treatment, affecting approximately one third of all patients who undergo chemotherapy (Argyriou et al.; 2012; Crit. Rev. Oncol. Hematol.; 28(1); 51-77). For several of the most effective drugs (e.g. taxanes, vinca alkaloids, cisplatin, bortezomib, thalidomide and lenolidamide), neurotoxicity is dose-limiting and sometimes forces the termination of otherwise successful therapy (Polomano and Bennett, Pain Med., 2001, 2(1), 8-14; Park et al., Curr. Med. Chem, 2008,15(29), 3081-94). Additionally, with the advent of effective treatments for ameliorating the neutropenia associated with chemotherapy, peripheral neuropathy is now one of the principal dose-limiting side effects of chemotherapeutic agents (Quasthoff and Hartung; 2002; J. Neurol.; 249(1); 9-17). It impairs functional capacity, compromises quality of life and may result in dose reduction or cessation of chemotherapy, with the subsequent reduction of efficacy. Therefore, the tolerability to chemotherapy may affect patient's survival.

In some countries, as the US, Colorectal cancer (CRC) is the fourth most frequently diagnosed cancer and the second leading cause of neoplasm-related death. Approximately 25% of patients already have metastatic disease at the moment of diagnosis and nearly 50% will develop metastases. Over the past 10 years, various combinations of chemotherapy were investigated for the treatment of metastatic colorectal cancer (m-CRC) based on Oxaliplatin (as 1^{st} priority), Vincristine, Paclitaxel (as 2^{nd} priority), Doxitaxel and Cisplatin.

Then, different combination therapies have been tested regarding the improvement of chemotherapy efficacy (enablement) with different even controversial results.

In addition to well-known classic agents, novel drugs such as oxaliplatin (OXA) have been also shown to bear a significant risk of peripheral neuropathy (Argyriou et al.; 2008; Cancer Treat. Rev.; 34(4); 368-377; Velasco and Bruna; 2010; Neurologia; 25(2); 116-131; Argyriou et al. 2012).

The introduction of Oxaliplatin as neoadjuvant concurrent in chemotherapy and/or radiotherapy represented an improvement, among others:
- for the standard care of LARC (Locally Advanced Rectal Cancer) represented by FU (5-Fluorouracil) and Capecitabine-based preoperative CRT (chemoradiotherapy).
- combined with 5-Fluorouracil/Leucovotin (5-FU/LV) and Capecitabine in the treatment of patients with stage III (Duke's C) colon cancer after complete surgical resection of the primary tumor as compared with adjuvant chemotherapy with an stablished fluorouracil-containing regimen (Pandor,A. et al., 2006, Health Technol. Assess., 10(41); Hind,D. et al., 2008, Health Technol. Assess., 12(15)).
- which is known as triplet chemotherapy: 5-Fluorouracil (5-FU)/Leucovorin (LV), Oxaliplatin and Irinotecan (FOLFOXIRI: FOLFOX combined with Irinotecan or FOLFIRINOX: FOLFIRI combined with Oxaliplatin) in combination with the vascular endothelial growth factor inhibitor Bevacizumab (Loupakis,F. et al., 2016, Targ. Oncol., 11, 293-308).

But in all above-mentioned studies it is also indicated that oxaliplatin-based regimens (OX/FU, FOLFOX4, FLOX, etc.) significantly increased 3-4 grade acute toxicities and/or adverse events compared with the non oxaliplatin-based ones.

As above-mentioned, Oxaliplatin, a third generation of platinum analogues, has been incorporated in combination as a first-line drug for the treatment of colorectal cancer during the last decade. In addition to colorectal cancer, Oxaliplatin is active in a range of malignancies (ovarian, non-Hodgkin lymphoma, breast cancer, mesothelioma, nonsmall cell lung cancer) and has undergone clinical development against gastric cancer (Culy et al., 2000, Drugs, 60(4), 895-924). Side effects frequently include hematologic toxicity (neutropenia, thrombocytopenia) and gastrointestinal toxicity (nausea, vomiting and diarrhea). However, its clinical use is commonly hampered by the occurrence of dose-limiting peripheral neuropathy. Oxaliplatin-induced neuropathy may result in dose reduction or cessation of chemotherapy, with the subsequent reduction of efficacy.

Chemotherapeutic techniques have a range of side effects, also known as adverse effects, which depend on the type of medications used. The most common medications affect mainly the fast-dividing cells of the body, such as blood cells and the cells lining the mouth, stomach, and intestines. Chemotherapy-related toxicities can occur acutely after administration, within hours or days, or chronically, from weeks to years.

Among others there have been described: Immunosuppression and myelosuppression, neutropenic enterocolitis, gastrointestinal distress, anemia, fatigue, nausea and vomiting, hair loss, secondary neoplasm, infertility, teratogenicity, peripheral neuropathy, cognitive impairment, tumor lysis syndrome and organ damage. The occurrence and severity of the neuropathy is dependent on single dose intensity, duration of treatment, cumulative dose, prior or concurrent treatment with other neuropathic drugs and co-existing conditions such as diabetes and alcohol abuse (Alberts et al., Anticancer Drugs, 1995, 6(3), 369-83; Postma et al., Ann. Oncol., 1995, 6(5), 489-94; Forsyth et al., J. Neurooncol., 1997, 35(1), 47-53; Quasthoff and Hartung, J. Neurol., 2002, 249(1), 9-17). The treatment of the very specific symptoms arising from the neurotoxicity of the chemotherapeutic drug is crucial for preserving the quality of life of the afflicted patients (Mielke et al., Eur. J. Cancer, 2006, 42(1), 24-30; Park et al., Curr. Med. Chem., 2008, 15(29), 3081-94; Argyriou et al., Blood, 2008, 112(5), 1593-9). Unfortunately, an effective treatment for chemotherapy-induced peripheral neuropathy has yet to be found (Wolf et al., Eur. J. Cancer, 2008, 44(11), 1507-15).

Therefore, there is still a need to improve the use of chemotherapy for treating cancer, in particular, a need for effective therapies to prevent and/or treat the Chemotherapy Induced Peripheral Neuropathy (CIPN) and/or a need for new therapies against cancer, increasing the efficacy of the chemotherapeutic drugs, by reducing the cancer cells proliferation or by diminishing the associated adverse effects or both.

WO 2010/128309 discloses anti-tumour synergy between rimcazole and paclitaxel in the treatment of mice bearing MDA-MB-231 breast cancer adenocarcinoma xenografts.

Wang et al., [Breast Cancer Res. Treat., 2004, 87:205-214] describes a significant additive growth-inhibiting effect of reduced haloperidol in MDA 361 breast tumour cells when combined with doxorubicin, paclitaxel, docetaxel and vinorelbine.

The sigma (σ) receptor is a cell surface and endoplasmic reticulum receptor expressed in the central nervous system (CNS) among other tissues. From studies of the biology and function of sigma receptors, evidence has been presented that sigma receptor ligands may be useful in the treatment of psychosis and movement disorders such as dystonia and tardive dyskinesia, and motor disturbances associated with Huntington's chorea or Tourette's syndrome and in Parkinson's disease (Walker, J.M. et al, Pharmacological Reviews, 1990, 42, 355). It has been reported that the known sigma receptor ligand rimcazole clinically shows effects in the treatment of psychosis (Hanner, M. et al., Proc. Natl. Acad. Sci., 1996, 93:8072-8077; Snyder, S.H., Largent, B.L., J. Neuropsychiatry ,1989, 1, 7). The sigma binding sites have preferential affinity for the dextrorotatory isomers of certain opiate benzomorphans, such as (+)SKF 10047, (+)cyclazocine, and (+)pentazocine and also for some narcoleptics such as haloperidol.

The sigma receptor has at least two subtypes, which may be discriminated by stereoselective isomers of these pharmacoactive drugs. SKF 10047 has nanomolar affinity for the sigma 1 (σ-1) site, and has micromolar affinity for the sigma (σ-2) site. Haloperidol has similar affinities for both subtypes. Endogenous sigma ligands are not known, although progesterone has been suggested to be one of them. Possible sigma-site-mediated drug effects include modulation of glutamate receptor function, neurotransmitter response, neuroprotection, behavior, and cognition (Quirion, R. et al., Trends Pharmacol. Sci., 1992, 13:85-86). The existence of sigma receptors in the CNS, immune and endocrine systems have suggested a likelihood that it may serve as link between the three systems.

Sigma receptors are overexpressed in a large variety of tumors. In vivo studies with sigma ligands may provide important information about the biology of sigma receptors and may lead to the application of PET imaging for tumor detection, tumor staging, evaluation of therapeutic strategies and antitumor drug development. Sigma ligands are potentially useful as anticancer drugs either for single agent or adjuvant chemotherapy (van Waarde et al.; Biochim. Biophys. Acta; 2015; 1848; 2703-2714).

The inventors found one family of pyrazole derivatives which are particularly selective inhibitors of the sigma-1 receptor. This family presents a pyrazole group which is characterized by the substitution at position 3 by an alkoxy group directly bounded to nitrogen. These compounds were described in WO 2006/021462.

The sigma-1 receptor ligands disclosed in WO 2006/021462 have shown to be effective in the management of chemotherapy induced pain (EP 2,292,236) and for preventing the emesis induced by chemotherapy (EP 2,388,005). However, to the best knowledge of the inventors there is no previous disclosure about the utility of these compounds as disclosed in the present invention.

### SUMMARY OF THE INVENTION

The invention is set out in the appended set of claims.

The inventors of the present invention have surprisingly found and demonstrated that the administration of the specific sigma-receptor ligands according to general formula (I) is highly effective for reducing cancer cells proliferation and/or increasing the efficacy of the chemotherapeutic agent when administered as adjuvant therapy. 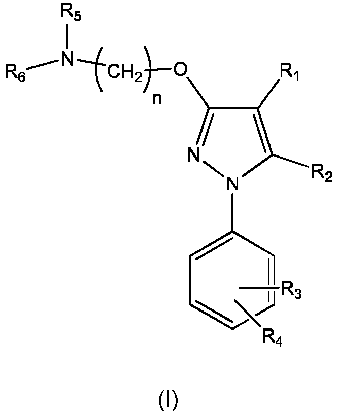 wherein
**R₁** is selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted non-aromatic heterocyclyl, substituted or unsubstituted aromatic heterocyclyl, substituted or unsubstituted heterocyclylalkyl, -COR₈, -C(O)OR₈, -C(O)NR₈R₉, -CH=NR₈, -CN, -OR₈, -OC(O)R₈, -S(O)ₜ-R₈, -NR₈R₉, -NR₈C(O)R₉, - NO₂, - N=CR₈R₉, and halogen;
**R₂** is selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, - COR₈, -C(O)OR₈, -C(O)NR₈R₉, -CH=NR₈, -CN, - OR₈, -OC(O)R₈, -S(O)ₜ-R₈, -NR₈R₉, -NR₈C(O)R₉, -NO₂, -N=CR₈R₉, and halogen;
**R₃** and **R₄** are independently selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, -COR₈, -C(O)OR₈, - C(O)NR₈R₉, -CH=NR₈, -CN, -OR₈, -OC(O)R₈, -S(O)ₜ-R₈, -NR₈R₉, -NR₈C(O)R₉, - NO₂, -N=CR₈R₉, and halogen, or together they form an optionally substituted fused ring system;
**R₅** and **R₆** are independently selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, -COR₈, -C(O)OR₈, - C(O)NR₈R₉, -CH=NR₈, -CN, -OR₈, -OC(O)R₈, -S(O)ₜ-R₈, -NR₈R₉, -NR₈C(O)R₉, - NO₂, -N=CR₈R₉, and halogen, or together form, with the nitrogen atom to which they are attached, a substituted or unsubstituted, aromatic or non-aromatic heterocyclyl group;
**n** is selected from 1, 2, 3, 4, 5, 6, 7 or 8;
**t** is 1,2 or 3;
**R₈** and **R₉** are each independently selected from hydrogen, substituted or unsubstituted alkyl, substituted or unsubstituted cycloalkyl, substituted or unsubstituted alkenyl, substituted or unsubstituted aryl, substituted or unsubstituted, aromatic or non-aromatic heterocyclyl, substituted or unsubstituted alkoxy, substituted or unsubstituted aryloxy, and halogen;
or a pharmaceutically acceptable salt, isomer, prodrug or solvate thereof.

The inventors have also found that the administration of these specific sigma receptor ligands is effective to prevent and/or treat the Chemotherapy Induced Peripheral Neuropathy (CIPN), particularly performing a neuroprotective role for chronic cumulative CIPN.

Even more surprisingly, it has been demonstrated that the co-administration of these sigma receptor ligands, as adjuvant therapy, with a chemotherapeutic drug enables chemotherapy allowing higher chemotherapy dosing, longer treatment with chemotherapy and less withdrawals which revers in an increase of patients' survival.

Consequently, there is an increment in the safety and tolerability of the chemotherapeutic drugs.

In particular, the invention is directed to the compound 4-{2-[5-methyl-1-(naphthalen-2-yl)-1H-pyrazol-3-yloxy]ethyl}morpholine or a pharmaceutically acceptable salt or solvate thereof for use in increasing the efficacy of a taxane chemotherapeutic drug by reducing cancer cells proliferation.

These aspects and preferred embodiments thereof are additionally also defined hereinafter in the detailed description and in the claims.

### BRIEF DESCRIPTION OF THE FIGURES

**Figure 1A****:** Proliferation inhibition (IC₅₀ value) of Paclitaxel (Taxol) for MCF7 European cell line
**Figure 1B**: Proliferation inhibition (IC₅₀ value) of Paclitaxel (Taxol) for A-549 cell line.
**Figure 1C**: Proliferation inhibition (IC₅₀ value) of Paclitaxel (Taxol) for B16 cell line.
**Figure 2**: Proliferation inhibition of Example 1 in different cancer cell lines.
**Figure 3**: Proliferation inhibition of B16-F10 cell line of association of Paclitaxel (Taxol) and sigma-1 receptor antagonists Haloperidol, BD-1063 and Example 1.
**Figure 4**: Proliferation inhibition of MCF7 European cell line of association of Paclitaxel (Taxol) and sigma-1 receptor antagonists Haloperidol, BD-1063 and Example 1.

### DETAILED DESCRIPTION OF THE INVENTION

The compounds of general formula (I) bind with high affinity to the sigma-receptor, and they are particularly selective inhibitors of the sigma-1 receptor subtype.

Particular examples of the sigma ligand of general formula (I) are:
[1] 4-{2-(1-(3,4-Dichlorophenyl)-5-methyl-1H pyrazol-3-yloxy)ethyl} morpholine,
[2] 2-[1-(3,4-Dichlorophenyl)-5-methyl-1H-pyrazol-3-yloxy]-N,N-diethylethanamine hydrochloride,
[3] 1-(3,4-Dichlorophenyl)-5-methyl-3-[2-(pyrrolidin-1-yl)ethoxy]-1H-pyrazole hydrochloride,
[4] 1-(3,4-Dichlorophenyl)-5-methyl-3-[3-(pyrrolidin-1-yl)propoxy]-1H-pyrazole hydrochloride,
[5] 1-{2-[1-(3,4-Dichlorophenyl)-5-methyl-1H-pyrazol-3-yloxy]ethyl}piperidine,
[6] 1-{2-[1-(3,4-dichlorophenyl)-5-methyl-1H-pyrazol-3-yloxy]ethyl}-1H-imidazole,
[7] 3-{1-[2-(1-(3,4-Dichlorophenyl)-5-methyl-1H-pyrazol-3-yloxy)ethyl]piperidin-4-yl}-3H-imidazo[4,5-b]pyridine,
[8] 1-{2-[1-(3,4-Dichlorophenyl)-5-methyl-1H-pyrazol-3-yloxy]ethyl}-4-methylpiperazine,
[9] Ethyl 4-{2-[1-(3,4-dichlorophenyl)-5-methyl-1H-pyrazol-3-yloxy]ethyl}piperazine carboxylate,
[10] 1-(4-(2-(1-(3,4-dichlorophenyl)-5-methyl-1H-pyrazol-3-yloxy)ethyl)piperazin-1-yl)ethanone,
[11] 4-{2-[1-(4-Methoxyphenyl)-5-methyl-1H-pyrazol-3-yloxy]ethyl}morpholine hydrochloride,
[12] 1-(4-Methoxyphenyl)-5-methyl-3-[2-(pyrrolidin-1-yl)ethoxy]-1H-pyrazole,
[13] 1-(4-Methoxyphenyl)-5-methyl-3-[3-(pyrrolidin-1-yl)propoxy]-1H-pyrazole,
[14] 1-[2-(1-(4-Methoxyphenyl)-5-methyl-1H-pyrazol-3-yloxy)ethyl]piperidine,
[15] 1-{2-[1-(4-Methoxyphenyl)-5-methyl-1H-pyrazol-3-yloxy]ethyl}-1H-imidazole,
[16] 4-{2-[1-(3,4-Dichlorophenyl)-5-phenyl-1H-pyrazol-3-yloxy]ethyl}morpholine hydrochloride,
[17] 1-(3,4-Dichlorophenyl)-5-phenyl-3-[2-(pyrrolidin-1-yl)ethoxy]-1H-pyrazole hydrochloride,
[18] 1-(3,4-Dichlorophenyl)-5-phenyl-3-[3-(pyrrolidin-1-yl)propoxy]-1H-pyrazole,
[19] 1-{2-[1-(3,4-Dichlorophenyl)-5-phenyl-1H-pyrazol-3-yloxy]ethyl}piperidine,
[20] 1-{2-[1-(3,4-Dichlorophenyl)-5-phenyl-1H-pyrazol-3-yloxy]ethyl}-1H-imidazole hydrochloride,
[21] 2-{2-[1-(3,4-dichlorophenyl)-5-phenyl-1H-pyrazol-3-yloxy]ethyl}-1,2,3,4-tetrahydroisoquinoline hydrochloride,
[22] 4-{4-[1-(3,4-Dichlorophenyl)-5-methyl-1H-pyrazol-3-yloxy]butyl}morpholine hydrochloride,
[23] 1-(3,4-Dichlorophenyl)-5-methyl-3-[4-(pyrrolidin-1-yl)butoxy]-1H-pyrazole,
[24] 1-{4-[1-(3,4-Dichlorophenyl)-5-methyl-1H-pyrazol-3-yloxy]butyl}piperidine hydrochloride,
[25] 1-{4-[1-(3,4-Dichlorophenyl)-5-methyl-1H-pyrazol-3-yloxy]butyl}-4-methylpiperazine dihydrochloride,
[26] 1-{4-[1-(3,4-Dichlorophenyl)-5-methyl-1H-pyrazol-3-yloxy]butyl}-1H-imidazole,
[27] 4-[1-(3,4-Dichlorophenyl)-5-methyl-1H-pyrazol-3-yloxy]-N,N-diethylbutan-1-amine,
[28] 1-{4-[1-(3,4-dichlorophenyl)-5-methyl-1H-pyrazol-3-yloxy]butyl}-4-phenylpiperidine hydrochloride,
[29] 1-{4-[1-(3,4-dichlorophenyl)-5-methyl-1H-pyrazol-3-yloxy]butyl}-6,7-dihydro-1H-indol-4(5H)-one,
[30] 2-{4-[1-(3,4-dichlorophenyl)-5-methyl-1H-pyrazol-3-yloxy]butyl}-1,2,3,4-tetrahydroisoquinoline,
[31] 4-{2-[1-(3,4-dichlorophenyl)-5-isopropyl-1H-pyrazol-3-yloxy]ethyl}morpholine hydrochloride,
[32] 2-[1-(3,4-Dichlorophenyl)-5-isopropyl-1H-pyrazol-3-yloxy]-N,N-diethylethanamine,
[33] 1-(3,4-Dichlorophenyl)-5-isopropyl-3-[2-(pyrrolidin-1-yl)ethoxy]-1H-pyrazole hydrochloride,
[34] 1-(3,4-Dichlorophenyl)-5-isopropyl-3-[3-(pyrrolidin-1-yl)propoxy]-1H-pyrazole hydrochloride,
[35] 1-{2-[1-(3,4-Dichlorophenyl)-5-isopropyl-1H-pyrazol-3-yloxy]ethyl}piperidine,
[36] 2-{2-[1-(3,4-dichlorophenyl)-5-isopropyl-1H-pyrazol-3-yloxy]ethyl}-1,2,3,4-tetrahydroisoqui-noline hydrochloride,
[37] 4-{2-[1-(3,4-dichlorophenyl)-1H-pyrazol-3-yloxy]ethyl}morpholine,
[38] 2-[1-(3,4-dichlorophenyl)-1H-pyrazol-3-yloxy] N,N-diethylethanamine,
[39] 1-(3,4-dichlorophenyl)-3-[2-(pyrrolidin-1-yl)ethoxy]-1H-pyrazole,
[40] 1-{2-[1-(3,4-dichlorophenyl)-1H-pyrazol-3-yloxy]ethyl}piperidine,
[41] 1-(3,4-dichlorophenyl)-3-[3-(pyrrolidin-1-yl)propoxy]-1H-pyrazole,
[42] 1-{2-[1-(3,4-Dichlorophenyl)-5-methyl-1H-pyrazol-3-yloxy]ethyl}piperazine dihydrochloride,
[43] 1-{2-[1-(3,4-Dichlorophenyl)-5-methyl-1H-pyrazol-3-yloxy]ethyl}pyrrolidin-3-amine,
[44] 4-{2-[1-(3,4-Dichlorophenyl)-4,5-dimethyl-1H-pyrazol-3-yloxy]ethyl}morpholine,
[45] 2-[1-(3,4-Dichlorophenyl)-4,5-dimethyl-1H-pyrazol-3-yloxy]-N,N-diethylethanamine hydrochloride,
[46] 1-(3,4-Dichlorophenyl)-4,5-dimethyl-3-[2-(pyrrolidin-1-yl)ethoxy]-1H-pyrazole hydrochloride,
[47] 1-(3,4-Dichlorophenyl)-4,5-dimethyl-3-[3-(pyrrolidin-1-yl)propoxy]-1H-pyrazole hydrochloride,
[48] 1-{2-[1-(3,4-Dichlorophenyl)-4,5-dimethyl-1H-pyrazol-3-yloxy]ethyl}piperidine,
[49] 4-{4-[1-(3,4-dichlorophenyl)-1H-pyrazol-3-yloxy]butyl}morpholine hydrochloride,
[50] (2S,6R)-4-{4-[1-(3,4-dichlorophenyl)-1H-pyrazol-3-yloxy]butyl}-2,6-dimethylmorpholine hydrochloride,
[51] 1-{4-[1-(3,4-Dichlorophenyl)-1H-pyrazol-3-yloxy]butyl}piperidine hydrochloride,
[52] 1-(3,4-Dichlorophenyl)-3-[4-(pyrrolidin-1-yl)butoxy]-1H-pyrazole hydrochloride,
[53] 4-[1-(3,4-dichlorophenyl)-1H-pyrazol-3-yloxy]-N,N-diethylbutan-1-amine oxalate,
[54] N-benzyl-4-[1-(3,4-dichlorophenyl)-1H-pyrazol-3-yloxy]-N-methylbutan-1-amine oxalate,
[55] 4-[1-(3,4-dichlorophenyl)-1H-pyrazol-3-yloxy]-N-(2-methoxyethyl)-N-methylbutan-1-amine oxalate,
[56] 4-{4-[1-(3,4-dichlorophenyl)-1H-pyrazol-3-yloxy]butyl}thiomorpholine oxalate,
[57] 1-[1-(3,4-Dichlorophenyl)-5-methyl-3-(2-morpholinoethoxy)-1H-pyrazol-4-yl]ethanone oxalate,
[58] 1-{1-(3,4-dichlorophenyl)-5-methyl-3-[2-(pyrrolidin-1-yl)ethoxy]-1H-pyrazol-4-yl}ethanone oxalate,
[59] 1-{1-(3,4-dichlorophenyl)-5-methyl-3-[2-(piperidin-1-yl)ethoxy]-1H-pyrazol-4-yl}ethanone oxalate,
[60] 1-{1-(3,4-dichlorophenyl)-3-[2-(diethylamino)ethoxy]-5-methyl-1H-pyrazol-4-yl}ethanone oxalate,
[61] 4-{2-[5-Methyl-1-(naphthalen-2-yl)-1H-pyrazol-3-yloxy]ethyl}morpholine,
[62] N,N-Diethyl-2-[5-methyl-1-(naphthalen-2-yl)-1H-pyrazol-3-yloxy]ethanamine,
[63] 1-{2-[5-Methyl-1-(naphthalen-2-yl)-1H-pyrazol-3-yloxy]ethyl}piperidine hydrochloride,
[64] 5-Methyl-1-(naphthalen-2-yl)-3-[2-(pyrrolidin-1-yl)ethoxy]-1H-pyrazole hydrochloride,
their salts, different alternative pharmaceutically acceptable salts, solvates or prodrugs.

The invention is drawn to the compound 4-{2-[5-methyl-1-(naphthalen-2-yl)-1H-pyrazol-3-yloxy]ethyl}morpholine or a pharmaceutically acceptable salt or solvate thereof for use in increasing the efficacy of a taxane chemotherapeutic drug by reducing cancer cells proliferation.

In a preferred embodiment of the use as defined above the compound is 4-{2-[5-methyl-1-(naphthalen-2-yl)-1H-pyrazol-3-yloxy]ethyl}morpholine hydrochloride or a solvate thereof.

"Alkyl" refers to a straight or branched hydrocarbon chain radical consisting of carbon and hydrogen atoms, containing no saturation, having one to eight carbon atoms, and which is attached to the rest of the molecule by a single bond, e. g., methyl, ethyl, n-propyl, i-propyl, n-butyl, t-butyl, n-pentyl, etc. Alkyl radicals may be optionally substituted by one or more substituents such as a aryl, halo, hydroxy, alkoxy, carboxy, cyano, carbonyl, acyl, alkoxycarbonyl, amino, nitro, mercapto, alkylthio, etc. If substituted by aryl it corresponds to an "arylalkyl or aralkyl" radical, such as benzyl and phenethyl.

"Alkenyl" refers to an alkyl radical having at least two carbon atoms and having one or more unsaturated bonds.

"Cycloalkyl" refers to a stable 3-to 10-membered monocyclic or bicyclic radical which is saturated or partially saturated, and which consist solely of carbon and hydrogen atoms, such as cyclohexyl or adamantyl. The cycloalkyl radicalmay be optionally substituted by one or more substituents such as alkyl, halo, hydroxy, amino, cyano, nitro, alkoxy, carboxy, alkoxycarbonyl, etc.

"Aryl" refers to single and multiple ring radicals, including multiple ring radicals that contain separate and/or fused aryl groups. Typical aryl groups contain from 1 to 3 separated or fused rings and from 6 to about 18 carbon ring atoms, such as phenyl, naphthyl, indenyl, fenanthryl or anthracyl radical. The aryl radical may be optionally substituted by one or more substituents such as hydroxy, mercapto, halo, alkyl, phenyl, alkoxy, haloalkyl, nitro, cyano, dialkylamino, aminoalkyl, acyl, alkoxycarbonyl, etc.

"Heterocyclyl" refers to a stable 3-to 15 membered ring radical which consists of carbon atoms and from one to five heteroatoms selected from the group consisting of nitrogen, oxygen, and sulfur, preferably a 4-to 8-membered ring with one or more heteroatoms, more preferably a 5-or 6-membered ring with one or more heteroatoms. It may be aromatic or not aromatic. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized ; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran, coumarine, morpholine; pyrrole, pyrazole, oxazole, isoxazole, triazole, imidazole, etc.

"Alkoxy" refers to a radical of the formula -ORa where Ra is an alkyl radical as defined above, e. g., methoxy, ethoxy, propoxy, etc.

"Amino" refers to a radical of the formula-NH₂, -NHRa or -NRaRb, optionally quaternized, wherein Ra and Rb is an alkyl radical as defined above, e. g., methoxy, ethoxy, propoxy, etc.

"Halo" or "hal" refers to bromo, chloro, iodo or fluoro.

"Fused ring system" refers to a polycyclic ring system that contains fused rings. Typically, the fused ring system contains 2 or 3 rings and/or up to 18 ring atoms. As defined above, cycloalkyl radicals, aryl radicals and heterocyclyl radicals may form fused ring systems. Thus, fused ring system may be aromatic, partially aromatic or not aromatic and may contain heteroatoms. A spiro ring system is not a fused-polycyclic by this definition, but fused polycyclic ring systems may themselves have spiro rings attached thereto via a single ring atom of the system. Examples of fused ring systems are, but are not limited to, adamantyl, naphthyl (e.g. 2-naphthyl), indenyl, fenanthryl, anthracyl, pyrenyl, benzimidazole, benzothiazole, etc.

Unless otherwise stated specifically in the specification, all the groups may be optionally substituted, if applicable. References herein to substituted groups in the compounds refer to the specified moiety that may be substituted at one or more available positions by one or more suitable groups, e. g., halogen such as fluoro, chloro, bromo and iodo ; cyano; hydroxyl ; nitro ; azido ; alkanoyl such as a C₁₋₆ alkanoyl group such as acyl and the like; carboxamido; alkyl groups including those groups having 1 to about 12 carbon atoms or from 1 to about 6 carbon atoms and more preferably 1-3 carbon atoms; alkenyl and alkynyl groups including groups having one or more unsaturated linkages and from 2 to about 12 carbon or from 2 to about 6 carbon atoms; alkoxy groups having one or more oxygen linkages and from 1 to about 12 carbon atoms or 1 to about 6 carbon atoms; aryloxy such as phenoxy; alkylthio groups including those moieties having one or more thioether linkages and from 1 to about 12 carbon atoms or from 1 to about 6 carbon atoms; alkylsulfinyl groups including those moieties having one or more sulfinyl linkages and from 1 to about 12 carbon atoms or from 1 to about 6 carbon atoms ; alkylsulfonyl groups including those moieties having one or more sulfonyl linkages and from 1 to about 12 carbon atoms or from 1 to about 6 carbon atoms; aminoalkyl groups such as groups having one or more N atoms and from 1 to about 12 carbon atoms or from 1 to about 6 carbon atoms; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

The term "pharmaceutically acceptable salts, solvates, prodrugs" refers to any pharmaceutically acceptable salt, ester, solvate, or any other compound which, upon administration to the recipient is capable of providing (directly or indirectly) a compound as described herein. However, it will be appreciated that non-pharmaceutically acceptable salts also fall within the scope of the invention since those may be useful in the preparation of pharmaceutically acceptable salts. The preparation of salts, prodrugs and derivatives can be carried out by methods known in the art.

For instance, pharmaceutically acceptable salts of compounds provided herein are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic aminoacids salts.

Particularly favored derivatives or prodrugs are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

Also disclosed are compounds that are a prodrug of a compound of formula (I), not covered by the invention. The term "prodrug" is used in its broadest sense and encompasses those derivatives that are converted in vivo to the compounds of formula (I). Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides. Examples of well-known methods of producing a prodrug of a given acting compound are known to those skilled in the art and can be found e.g. in Krogsgaard-Larsen et al. "Textbook of Drug design and Discovery" Taylor & Francis (april 2002).

The compounds of the invention may be in crystalline form either as free compounds or as solvates and it is intended that both forms are within the scope of the present invention. Methods of solvation are generally known within the art. Suitable solvates are pharmaceutically acceptable solvates. In a particular embodiment, the solvate is a hydrate.

The compound 4-{2-[5-Methyl-1-(naphthalen-2-yl)-1H-pyrazol-3-yloxy]ethyl}morpholine or its salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment, it is above 95% of 4-{2-[5-Methyl-1-(naphthalen-2-yl)-1H-pyrazol-3-yloxy]ethyl}morpholine, or of its salts or solvates.

The compounds represented by the above-described general formula (I) may include enantiomers depending on the presence of chiral centers or isomers depending on the presence of multiple bonds (e.g. Z, E).

The compound 4-{2-[5-Methyl-1-(naphthalen-2-yl)-1H-pyrazol-3-yloxy]ethyl}morpholine and its salts or solvates can be prepared as disclosed in the previous application WO 2006/021462.

The obtained reaction products may, if desired, be purified by conventional methods, such as crystallization and chromatography. Where the above-described processes for the preparation of compounds of the invention give rise to mixtures of stereoisomers, these isomers may be separated by conventional techniques such as preparative chromatography. If there are chiral centers the compounds may be prepared in racemic form, or individual enantiomers may be prepared either by enantiospecific synthesis or by resolution.

One preferred pharmaceutically acceptable form is the crystalline form, including such crystalline form in pharmaceutical composition. In the case of salts and solvates, the additional ionic and solvent moieties must also be non-toxic. The compounds of the invention may present different polymorphic forms, it is intended that the invention encompass all such forms.

The preferred salts for 4-{2-[5-Methyl-1-(naphthalen-2-yl)-1H-pyrazol-3-yloxy]ethyl}morpholine are disclosed in the previous application WO 2011/064315.

The preferred polymorphic forms for 4-{2-[5-Methyl-1-(naphthalen-2-yl)-1H-pyrazol-3-yloxy]ethyl}morpholine are disclosed in the previous application WO 2011/095579.

As used herein the term "chemotherapy" or "chemotherapeutic drug" refers broadly to the use of chemical drugs for the treatment of cancer, tumors or malign neoplasia.

"Plant alkaloids" (and terpenoids) are alkaloids derived from plants that block cell division by preventing microtubule function. Since microtubules are vital for cell division, their inhibition also arrests cell mitosis. The main examples of plant alkaloids are vinca alkaloids and taxanes.

"Vinca alkaloids" bind to specific sites on tubulin, inhibiting the assembly of tubulin into microtubules (M phase of the cell cycle). They are derived from the Madagascar periwinkle, Catharanthus roseus (formerly known as Vinca rosea). The preferred vinca alkaloids include Vincristine, Vinblastine, Vinorelbine, and Vindesine.

"Taxanes" are derived from the Pacific yew tree, Taxus brevifolia. Taxanes enhance the stability of microtubules, preventing the separation of chromosomes during anaphase. Preferred taxanes in this invention include Paclitaxel and Docetaxel.

Thus, examples of chemotherapeutic drug include taxanes, vinca alkaloids, drugs derived from platinum, from plant alkaloids, bortezomib, thalidomide and its derivatives or terpenes (terpenoids). More particular examples of chemotherapeutic drug include paclitaxel, oxaliplatin, cisplatin, carboplatin, vincristine, bortezomib, thalidomide or lenolidamide,

In the invention, the chemotherapeutic drug is a taxane, particularly Paclitaxel. Paclitaxel (Taxol^{®}) is one of the most effective and commonly used antineoplasic drugs for the treatment of solid tumours. It has two serious side effects, myelosupression and peripheral neurotoxicity. The granulocyte colony-stimulating factor effectively counteracts the neutropenia in most patients. However, there are no acceptable therapies to prevent or minimize the nerve damage, making neurotoxicity a significant dose-limiting side effect (Rowinsky et al., Semin. Oncol., 1993a 29 4 Suppl. 3, 1-15; Rowinsky et al., J. Clin. Oncol., 1993b, 11(10), 2010-20; Wasserheit et al., J. Clin. Oncol., 1996, 14(7), 1993-9; Gordon et al., J. Clin. Oncol., 1997, 15(5), 1965-73; Mielke et al., Eur. J. Cancer, 2006, 42(1), 24-30).

In another disclosure not covered by the invention, the chemotherapeutic drug is a platinum-containing drug, particularly Oxaliplatin or Cisplatin. These platinum-based chemotherapies are a mainstay for the treatment of solid tumors, especially colorectal, but also ovarian, testicular, bladder and lung cancer, but their clinical use is severely curtailed by dose-limiting neurotoxicity.

Colorectal cancer is one of the leading causes of cancer-related death in Western world.

As previously disclosed, Oxaliplatin-based regimens have become the standard therapy for locally advanced colorectal cancer. Oxaliplatin (OXA), a third-generation of platinum analogues, is very effective for the treatment of colorectal cancer (Andre et al.; 2004, Andre et al.; 2009). However, its clinical use is commonly hampered, among others, by the occurrence of dose-limiting peripheral neuropathy (Cersosimo; 2005).

Chemotherapy schedules usually used in colorectal cancer include combinations of 5-Fluoracil (5FU), Leucovorin (LV) and Oxaliplatin (FOLFOX) or Capecitabine, Leucovorin and Oxaliplatin (XELOX) in non-metastatic cancer. In metastatic disease, chemotherapy regimens with 5FU/LV and Oxaliplatin or Irinotecan now represent standard of care chemotherapy backbones in both, 1^{st} and 2^{nd} line therapy. However, the efficacy of these cytotoxic combinations is limited and the outcomes are mixed. Newly approved biological treatments involve antibodies against the Epidermal Growth Factor Receptor (EGFR), Cetuximab and Panitumumab, for the population of patients that have RAS wild-type tumors, VEGF-binding antibodies or other VEGF-trapping therapies, including Bevacizumab and Aflibercept, as well as Regorafenib, a multiple tyrosine kinase inhibitor.

But in all the above-mentioned studies it is also indicated that oxaliplatin-based regimens (OX/FU, FOLFOX4, FLOX etc) significantly increased 3-4 grade acute toxicities and/or adverse events compared with the non oxaliplatin-based ones.

Particularly, Oxaliplatin treatment results in two forms of neurotoxicity: acute and chronic. The acute form occurs in >90% of patients and may begin during the infusion or within hours of completion, is usually self-limited, and may be exacerbated by exposure to cold. Chronic neuropathy is cumulative and is most commonly seen in patients who have received total doses ≥540 mg/m². Although it is a sensory neuropathy, the intensity can increase to the point that it impairs physical functions, such as holding objects and writing (Cersosimo RJ., Ann. Pharmacother., 2005, 39(1), 128-135).

The conventional chemotherapy protocol in adjuvant setting is the schedule named FOLFOX 4, which consists in a 2-hour infusion of Leucovorin (200 mg/m²) followed by a 5-Fluorouracil bolus (400 mg/m²) and a 22-hour infusion (600 mg/m²/day) for 2 consecutive days every 2 weeks, together with Oxaliplatin 85 mg/m² as a 2-hour infusion on Day 1 (Andre et al.; 2004). In patients with metastatic cancer, the modified FOLFOX 6 schedule is usually administered, which consists of a 2-hour infusion of Leucovorin (400 mg/m²) followed by a 5-Fluorouracil bolus (400 mg/m²) and a 46-hour infusion (2400 mg/m²) every 2 weeks, together with Oxaliplatin 85 mg/m² as a 2-hour infusion on Day 1 (Maindrault-Goebel et al.; 2000). Routine antiemetic prophylaxis in addition to Dexamethasone are usually employed in both schedules.

Several agents to reduce or ameliorate the OXA-induced neuropathy have been evaluated with conflicting results. Therefore, there is neither a widely accepted strategy to ameliorate the symptoms of acute OXA-induced neuropathy nor established medical therapies to prevent or treat chronic OXA-induced neuropathy. In summary, data are currently insufficient for recommending any neuroprotective agent for the treatment of OXA-induced neuropathy (Velasco and Bruna; 2010).

Other adverse effects of Oxaliplatin may include fatigue; nausea, vomiting or diarrhea; neutropenia (low number of a type of white blood cells); ototoxicity (hearing loss); extravasation (if Oxaliplatin leaks from the infusion vein it may cause severe damage to the connective tissues); hypokalemia (low blood potassium, more common in womwns than men), persistent hiccups and rhabdomyolysis. Although, Oxaliplatin has less ototoxicity and nephrotoxicity than Cisplatin and Carboplatin.

Chemotherapy-induced peripheral neuropathy (CIPN) is the most prevalent neurological complication of anti-cancer treatment, affecting approximately one-third of all patients who undergo chemotherapy (Argyriou et al.; 2011).

As used herein, the terms "treat", "treating" and "treatment" include the eradication, removal, reversion, alleviation, modification, or control of CIPN and/or other adverse effects induced by chemotherapy. In certain embodiments, the terms "treat", "treating" and "treatment" relate to reducing the cancer cells proliferation.

As used herein, the terms "prevention", "preventing", "preventive" "prevent" and "prophylaxis" refers to the capacity of a therapeutic to avoid, minimize or difficult the onset or development of a disease or condition before its onset, in this case CIPN and/or other adverse effects induced by chemotherapy. Particularly, it includes the use of a therapeutic as a protection against CIPN and for slowing the onset. In certain embodiments, the terms "prevention", "preventing", "preventive" "prevent" and "prophylaxis" relate to reducing the cancer cells proliferation.

4-{2-[5-methyl-1-(naphthalen-2-yl)-1H-pyrazol-3-yloxy]ethyl}morpholine or a pharmaceutically acceptable salt or solvate thereof increases the efficacy of a taxane chemotherapeutic drug by reducing cancer cells proliferation

Therefore, 4-{2-[5-methyl-1-(naphthalen-2-yl)-1H-pyrazol-3-yloxy]ethyl}morpholine or a pharmaceutically acceptable salt or solvate thereof may be useful as an agent in ameliorating adverse events associated with taxane chemotherapy.

Particularly, 4-{2-[5-methyl-1-(naphthalen-2-yl)-1H-pyrazol-3-yloxy]ethyl}morpholine or a pharmaceutically acceptable salt or solvate thereof may be useful as a neuroprotective agent to prevent and/or treat Chemotherapy Induced Peripheral Neuropathy (CIPN) associated with taxanes.

4-{2-[5-Methyl-1-(naphthalen-2-yl)-1H-pyrazol-3-yloxy]ethyl}morpholine or a pharmaceutically acceptable salt or solvate thereof and, at least, a taxane chemotherapeutic drug may be combined for simultaneous, separate or sequential administration.

As used herein the term "chemotherapy enablement" refers broadly to allow higher chemotherapy dosing, longer treatment with chemotherapy and less withdrawals which revers in an increase of patients' survival.

4-{2-[5-Methyl-1-(naphthalen-2-yl)-1H-pyrazol-3-yloxy]ethyl}morpholine or a pharmaceutically acceptable salt or solvate thereof and, at least, a taxane chemotherapeutic drug may be combined for simultaneous, separate or sequential administration, for use in chemotherapy enablement, by allowing higher chemotherapy dosing, longer treatment with chemotherapy and less withdrawals which revers in an increase of patients survival.

Preferred combinations comprise the combination of 4-{2-[5-Methyl-1-(naphthalen-2-yl)-1H-pyrazol-3-yloxy]ethyl}morpholine or a pharmaceutically acceptable salts or solvates thereof with Paclitaxel.

More preferred combinations comprise the combination of 4-{2-[5-Methyl-1-(naphthalen-2-yl)-1H-pyrazol-3-yloxy]ethyl}morpholine hydrochloride or solvates thereof with Paclitaxel.

In additional preferred embodiments, the preferences described above for the 4-{2-[5-Methyl-1-(naphthalen-2-yl)-1H-pyrazol-3-yloxy]ethyl}morpholine above as well as for the taxane chemotherapeutic drug are combined.

The combination of the invention may be formulated for its simultaneous, separate or sequential administration, with at least a pharmaceutically acceptable carrier, additive, adjuvant or vehicle. This has the implication that the combination of the two active compounds may be administered:
- as a combination that is being part of the same medicament formulation, the two active compounds being then administered always simultaneously.
- as a combination of two units, each with one of the active substances giving rise to the possibility of simultaneous, sequential or separate administration.

In a particular embodiment, 4-{2-[5-Methyl-1-(naphthalen-2-yl)-1H-pyrazol-3-yloxy]ethyl}morpholine or a pharmaceutically acceptable salt or solvate thereof is independently administered from the taxane chemotherapeutic drug (i.e in two units) but at the same time.

In another particular embodiment, 4-{2-[5-Methyl-1-(naphthalen-2-yl)-1H-pyrazol-3-yloxy]ethyl}morpholine or a pharmaceutically acceptable salt or solvate thereof is administered first, and then the taxane chemotherapeutic drug is separately or sequentially administered.

In yet another particular embodiment, the taxane chemotherapeutic drug is administered first, and then 4-{2-[5-Methyl-1-(naphthalen-2-yl)-1H-pyrazol-3-yloxy]ethyl}morpholine or a pharmaceutically acceptable salt or solvate thereof is administered, separately or sequentially, as defined.

The present invention further provides pharmaceutical compositions comprising 4-{2-[5-Methyl-1-(naphthalen-2-yl)-1H-pyrazol-3-yloxy]ethyl}morpholine, or a pharmaceutically acceptable salt, derivative, or stereoisomers thereof together with a pharmaceutically acceptable carrier, adjuvant, or vehicle, for administration to a patient.

Examples of pharmaceutical compositions include any solid (tablets, pills, capsules, granules etc.) or liquid (solutions, suspensions or emulsions) composition for oral, topical or parenteral administration.

In a preferred embodiment, the pharmaceutical compositions are in oral form, either solid or liquid. Suitable dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

In a preferred embodiment of the invention 4-{2-[5-Methyl-1-(naphthalen-2-yl)-1H-pyrazol-3-yloxy]ethyl}morpholine or a pharmaceutically acceptable salt or solvate thereof, optionally in the form of a pharmaceutical composition, are administered once daily.

In a preferred embodiment of the invention, 4-{2-[5-Methyl-1-(naphthalen-2-yl)-1H-pyrazol-3-yloxy]ethyl}morpholine or a pharmaceutically acceptable salt or solvate thereof is administered as a daily dose of from to 100 mg to 600 mg per day. Even more preferably, 4-{2-[5-Methyl-1-(naphthalen-2-yl)-1H-pyrazol-3-yloxy]ethyl}morpholine or a pharmaceutically acceptable salt or solvate thereof is administered as a daily dose of from 200 mg to 400 mg per day.

The compounds and compositions of this invention may be used to provide with other drugs a combination therapy. The other drugs may form part of the same composition, or be provided as a separate composition for administration at the same time or at different time.

Chemotherapy enablement is reflected by allowing higher chemotherapy dosing, longer treatment with chemotherapy and less withdrawals which revers in an increase of patients' survival.

Thus, in a preferred embodiment of the invention, the combination is prepared for the administration in each chemotherapy cycle wherein:
- 4-{2-[5-Methyl-1-(naphthalen-2-yl)-1H-pyrazol-3-yloxy]ethyl}morpholine or a pharmaceutically acceptable salt, isomer or solvate thereof is administered as a daily dose of from 100 mg to 600 mg per day during the first 5 days and
- the taxane is administered as a daily dose of from ≥60 mg/m² on day 1 of the cycle.

Even in a more preferably embodiment of invention, the combination is prepared for the administration in each chemotherapy cycle wherein:
- 4-{2-[5-Methyl-1-(naphthalen-2-yl)-1H-pyrazol-3-yloxy]ethyl}morpholine or a pharmaceutically acceptable salt, isomer or solvate thereof is administered as a daily dose of from 200 mg to 400 mg per day during the first 5 days and
- the taxane is administered as a daily dose of from ≥80 mg/m² on day 1 of the cycle.

Even in a still more preferably embodiment of invention, the combination is prepared for the administration in each chemotherapy cycle wherein:
- 4-{2-[5-Methyl-1-(naphthalen-2-yl)-1H-pyrazol-3-yloxy]ethyl}morpholine or a pharmaceutically acceptable salt, isomer or solvate thereof is administered as a daily dose of 400 mg per day during the first 5 days and
- the taxane is administered as a daily dose of 85 mg/m² on day 1 of the cycle.

The following examples are given only as further illustration of the invention; they should not be taken as a definition of the limits of the invention.

### EXAMPLES

### Example 1

### Synthesis of 4-{2-[5-Methyl-1-(naphthalen-2-yl)-1H-pyrazol-3-yloxy]ethyl} morpholine (compound 61) and its hydrochloride salt

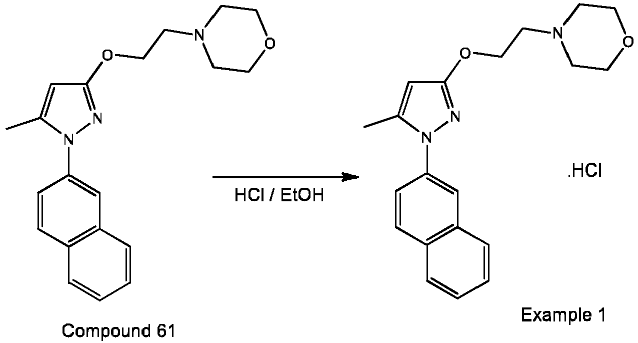

Compound 61 can be prepared as disclosed in the previous application WO2006/021462. Its hydrochloride can be obtained according the following procedure:
Compound 63 (6.39 g) was dissolved in ethanol saturated with HCl, the mixture was stirred then for some minutes and evaporated to dryness. The residue was crystallized from isopropanol. The mother liquors from the first crystallization afforded a second crystallization by concentrating. Both crystallizations taken together yielded 5.24 g (63 %) of the corresponding hydrochloride salt (m.p. = 197-199 °C).

1H-NMR (DMSO-d6) 6 ppm: 10,85 (bs, 1H), 7,95 (m, 4H), 7,7 (dd, J=2,2, 8,8 Hz,1H), 7,55 (m, 2H), 5,9 (s, 1H), 4,55 (m, 2H), 3,95 (m, 2H), 3,75 (m, 2H), 3,55-3,4 (m, 4H), 3,2 (m, 2H), 2,35 (s, 3H).

HPLC purity: 99.8%.

### Pharmacological data

### Materials and methods

### Cell lines cultures

The human breast adenocarcinoma MCF7 European cell line, the human lung carcinoma A549 cell line and the B16-F10 murine melanoma cell line were obtained from the University of Granada Scientific Instrumentation Centre (Spain) and the American Type Culture Collection (ATCC).The cells were cultured in Dulbecco's modified Eagle's medium (DMEM) (Sigma, St. Louis, MO, USA), supplemented with 10% heat-inactivated fetal bovine serum (FBS) (Lonza, Walkersville, MD, USA) and 1% of antibiotics mixture of penicillin (10,000 U/mL) and streptomycin (10 mg/mL) in an humidified 5% CO₂ incubator at 37 °C of temperature. Cultured cells were detached with a trypsin-ethylenediamine tetraacetic acid (EDTA) solution (0.25%) and seeded into 24-well plates at a density of 6-15 × 10³ cells per well, depending on the cell line.

### Proliferation assays

After incubation for 24 hours under culture conditions, the cells were treated with either paclitaxel (0.1-50 nM) or Example 1 (5-100 µM). When paclitaxel and the sigma-1 receptor antagonists were associated a concentration of paclitaxel (7.5-10 nM depending on the cell line) that produced 25-35% cytotoxicity was associated with 10 µM of each sigma-1 receptor antagonist (Example-1, BD-1063 or haloperidol). The percentage of viability was calculated by utilizing cells without any treatment as control.

After an incubation time of 96 hours with drugs, the cytotoxicity of the treatments toward the cell lines was evaluated in triplicate by the 3-(4,5-dimethylthiazol-2-yl)-2,5-diphenyl tetrazolium bromide (MTT) proliferation assay, as previously described (Clares et al., 2013), or with a sulforhodamine B (SRB) protocol, as previously described (Melguizo et al., 2015). In the MTT protocol, 20 µL of MTT solution in cell culture medium (5 mg/mL) was added to each well. After incubation during 4 h at this temperature, the culture medium was removed, and the resultant formazan crystals were dissolved in 200 µL of dimethylsulfoxide (DMSO). The optical density (OD) of converted dye, which is proportional to the number of viable cells, was measured at 570 nm using a Titertek multiscan colorimeter (Flow Laboratories, Irvine, USA). The percentage of surviving cells was calculated as the OD ratio of treated cells to control (untreated) cells (×100). In the SRB protocol, the cells were washed three times with PBS, and 300 µL of 10% trichloroacetic acid was used to fix the cells (20 minutes at 4°C). Then, the cells were washed three times with distilled water and left to dry before 300 µL of SRB was added. Incubation under mechanical stirring was continued for 20 minutes, and the excess SRB was removed by the addition of a 1% acetic acid solution. Then, the cells were left to dry. Finally, 200 µL of Trizma^{®} (10 mM, pH 10.5; Sigma-Aldrich) was used for dye resuspension. The optical density (OD) of SRB was measured at 492 nm (Titertek^{®} Multiskan colorimeter; Flow Laboratories Ltd, Oldham, UK) and analyzed with Ascent software version 2.6 (ThermoLab systems, Helsinki, Finland). In all cases, the percentage of surviving cells was calculated as: [OD treated cells/OD control (untreated) cells)] × 100. The IC₅₀ values (concentration of drug that inhibited 50% of cell proliferation) were estimated from the inhibition curves.

### Example 2: Cytotoxicity of paclitaxel (Taxol) and Example-1

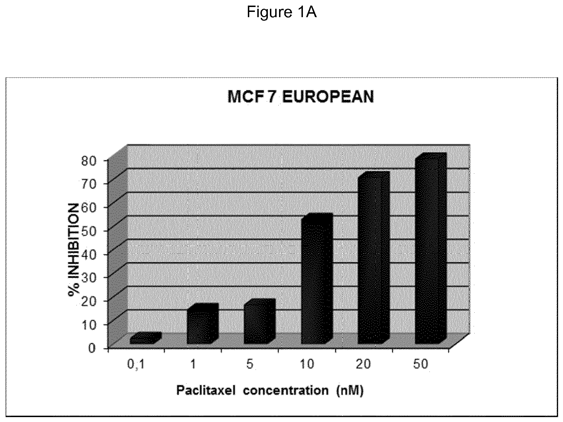

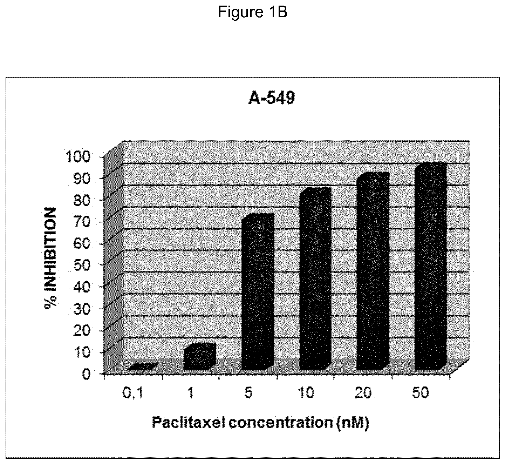

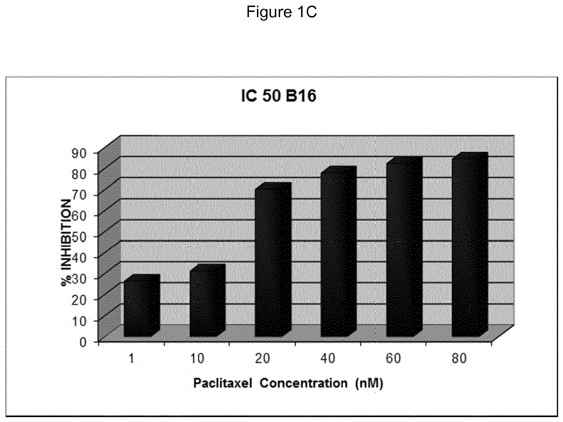

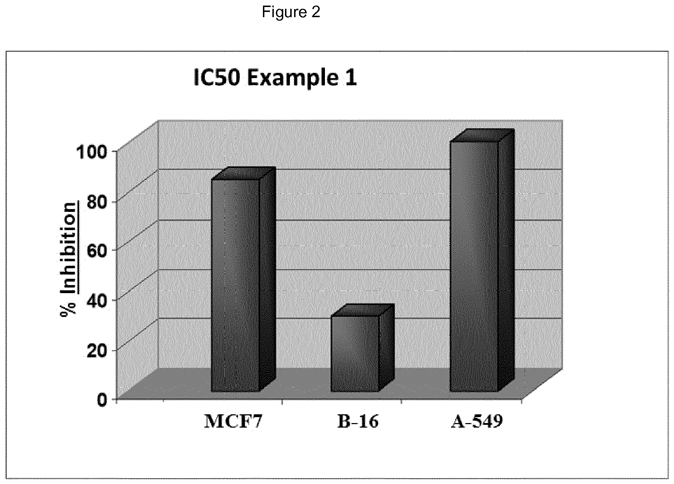

Paclitaxel produced a concentration-dependent cytotoxicity in all cell lines, with IC₅₀ values of 8.5, 3 and 15 nM for MCF7 European, A-549 and B16-F10 cells, respectively (Figure 1A, 1B and 1C). Example 1 also produced a concentration-dependent cytotoxicity in all cell lines, with IC₅₀ values of 83, 100 and 27 µM for MCF7 European, A-549 and B16-F10 cells, respectively (Figure 2).

### Example 3: Association of paclitaxel (Taxol) and sigma-1 receptor antagonists

In this example, the association of paclitaxel with example-1 falls under the claimed invention. The association of paclitaxel with BD-1063 and haloperidol is not encompassed by the wording of the claims but is considered as useful for understanding the invention.

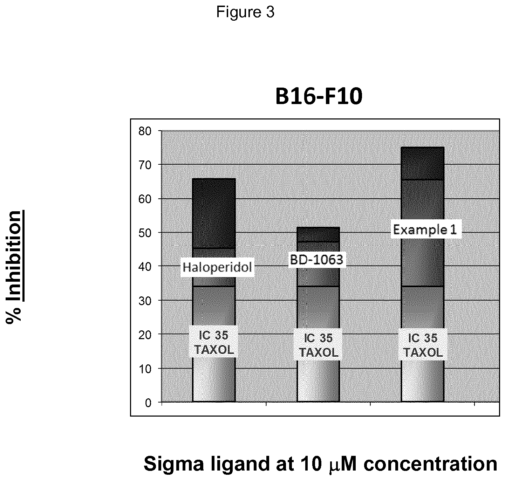

When a low concentration (10 nM) of paclitaxel that produce around 35% of cytotoxicity was associated with a low concentration (10 µM) of Example-1, BD-1063 or haloperidol in B16-F10 cell culture the cytotoxic effect obtained was greater than summative in all cases (Figure 3).

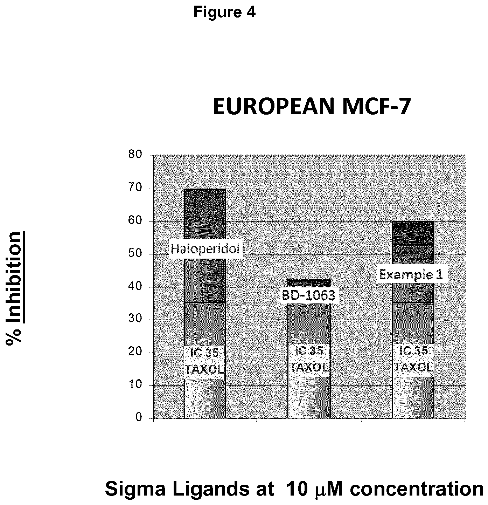

When a low concentration (10 nM) of paclitaxel (Taxol) that produce around 35% of cytotoxicity was associated with a low concentration (10 µM) of Example-1, BD-1063 or haloperidol in MCF7 European cell culture the cytotoxic effect obtained was greater than summative in all cases (Figure 4).

In summary, the results obtained in example 2 and example 3 indicate that proliferation of all cell lines studied is inhibited by the sigma-1 receptor antagonist tested and that sigma-1 receptor antagonists are able to increase the antiproliferative effect of paclitaxel.

It is also proved that Example 1 obtained better results than Haloperidol and BD-1063 in both cases.

### Clinical Data

### Example 4: Association of Oxaliplatin and a Sigma receptor antagonist, example 1, in patients treated for colorectal cancer (not encompassed by the claimed invention).

The results obtained in Example 3 were corroborated through the related parameters measurement in the corresponding clinical study done with the Example 1, as can be seen in Table 1.

Thus, the clinical study titled "A proof-concept phase 2, randomized, placebocontrolled, double blind, multicenter clinical trial in two parallel groups to evaluate the efficacy of Example 1 for reducing the incidence and severity of oxaliplatin-induced peripheral neuropathy in patients treated for colorectal cancer" was performed.

The study period was 24 weeks of chemotherapy (one chemo cycle every 2 weeks = 12 chemo cycles) plus 6 weeks (follow-up) and having as relevant safety objective to explore the incidence of adverse events by severity, of serious adverse effects, of adverse events leading to Example 1 discontinuation and of adverse events related to Example 1.

Patients received either:
- oral daily Example 1, 400 mg/day, during the first 5 days of every chemotherapy cycle, starting the day before the cycle, up to a maximum of 12 cycles (Arm 1),
- or oral daily placebo during the first 5 days of every chemotherapy cycle, starting the day before the cycle, up to a maximum of 12 cycles (Arm 2).

Regarding the chemotherapy received, all patients received Oxaliplatin as part of FOLFOX4 or modified FOLFOX6 chemotherapy schedule for the treatment of colorectal cancer. Patients were randomized to one of the two above mentioned Arms.

FOLFOX conditions were:
- **FOLFOX4** : oxaliplatin 85 mg/m² IV infusion on day 1, then leucovorin 200 mg/m² (or equivalent) IV infusion, then 5-FU 400 mg/m 2 IV bolus and 600 mg/m²/day during 22-hour continuous IV infusion on days 1 and 2; repeat every 2 wk for 12 cycles (6 months)
- **mFOLFOX6**: Oxaliplatin 85 mg/m² IV over 2 h on day 1 plus leucovorin 400 mg/m² IV over 2 h on day 1 plus 5-FU 400 mg/m² IV bolus on day 1, then 1200 mg/m²/day during 46 hours for 2 d continuous IV infusion; repeat every 2 wk for 12 cycles (6 months)

It was expected that chemotherapy cycles were repeated every 14 days for a maximum of 6 months (12 cycles) in the absence of disease progression or unacceptable toxicity. A follow-up visit was done about 6 weeks after the end of chemotherapy (the last cycle containing an Oxaliplatin dose ≥60 mg/m²).

The results of Table 1 were obtained:

**Table 1**

| **All Randomized Subjects** | **Example 1 (n=62) (%)** | **Placebo (n=62) (%)** |
|---|---|---|
| **Number of Completers** | **35 (56.5%)** | **26 (41.9%)** |
| **Number of discontinuations** | **27 (43.5%)** | **36 (51.8%)** |
| Progressive Disease | 2 (7.4%) | 9 (25.0%) |
| Physician decision | 3 (11.1%) | 9 (25.0%) |

Thus, the following conclusions were obtained:
- Patients treated with Example 1 attained higher Oxaliplatin dosing.
- Less patients treated with Example 1 discontinued because of cancer progression or because the decision of the physician.
- When example 1 was added to the chemotherapy, 14.6% more patients completed the 12 cycles of Oxaliplatin therapy.
- Patients treated with Example 1 tolerated better the chemotherapy.

## Claims

1. Compound 4-{2-[5-Methyl-1-(naphthalen-2-yl)-1H-pyrazol-3-yloxy]ethyl}morpholine or a pharmaceutically acceptable salt or solvate thereof for use in a therapeutic method of increasing the efficacy of a taxane chemotherapeutic drug by reducing cancer cells proliferation.

2. The compound for use according to claim 1, wherein the compound is 4-{2-[5-Methyl-1-(naphthalen-2-yl)-1H-pyrazol-3-yloxy]ethyl}morpholine hydrochloride or a solvate thereof.

3. The compound for use according to any one of claims 1 or 2, wherein the taxane is Paclitaxel.

4. The compound for use according to any one of claims 1 to 3, wherein the compound is administered as a daily dose of from to 100 mg to 600 mg per day.

5. The compound for use according claim 4, wherein the compound is administered as a daily dose of from to 200 mg to 400 mg per day.

6. The compound for use according to any one of claims 1 to 5, wherein the taxane forms part of the same composition as the compound.

7. The compound for use according to any one of claims 1 to 5, wherein the taxane is provided as a separate composition from the compound for administration at the same time or at different time.

8. The compound for use according to any one of claims 1 to 4, wherein:
• 4-{2-[5-Methyl-1-(naphthalen-2-yl)-1H-pyrazol-3-yloxy]ethyl}morpholine or a pharmaceutically acceptable salt or solvate thereof is administered at a daily dose of from 100 mg to 600 mg per day during the first 5 days, and
• the taxane is administered at a daily dose of at least ≥60 mg/m² on day 1 of the cycle.

9. The compound for use according to any one of claims 1 to 5, wherein:
• 4-{2-[5-Methyl-1-(naphthalen-2-yl)-1H-pyrazol-3-yloxy]ethyl}morpholine or a pharmaceutically acceptable salt, isomer or solvate thereof is administered at a daily dose of from 200 mg to 400 mg per day during the first 5 days, and
• the taxane is administered at a daily dose of from ≥80 mg/m² on day 1 of the cycle.

10. The compound for use according to any one of claims 1 to 5, wherein:
• 4-{2-[5-Methyl-1-(naphthalen-2-yl)-1H-pyrazol-3-yloxy]ethyl}morpholine or a pharmaceutically acceptable salt, isomer or solvate thereof is administered at a daily dose of 400 mg per day during the first 5 days, and
• the taxane is administered at a daily dose of from 85 mg/m² on day 1 of the cycle.

## Patentansprüche

1. Verbindung 4-{2-[5-Methyl-1-(naphthalen-2-yl)-1H-pyrazol-3-yloxy]ethyl}morpholin oder ein pharmazeutisch akzeptables Salz oder Solvat davon zur Verwendung in einem therapeutischen Verfahren zur Erhöhung der Wirksamkeit eines Taxan-Chemotherapeutikums durch Verringerung der Proliferation von Krebszellen.

2. Verbindung zur Verwendung nach Anspruch 1, wobei die Verbindung 4-{2-[5-Methyl-1-(naphthalen-2-yl)-1H-pyrazol-3-yloxy]ethyl}morpholinhydrochlorid oder ein Solvat davon ist.

3. Verbindung zur Verwendung nach einem der Ansprüche 1 oder 2, wobei das Taxan Paclitaxel ist.

4. Verbindung zur Verwendung nach einem der Ansprüche 1 bis 3, wobei die Verbindung als eine Tagesdosis von 100 mg bis 600 mg pro Tag verabreicht wird.

5. Verbindung zur Verwendung nach Anspruch 4, wobei die Verbindung als eine Tagesdosis von 200 mg bis 400 mg pro Tag verabreicht wird.

6. Verbindung zur Verwendung nach einem der Ansprüche 1 bis 5, wobei das Taxan einen Teil der gleichen Zusammensetzung wie die Verbindung bildet.

7. Verbindung zur Verwendung nach einem der Ansprüche 1 bis 5, wobei das Taxan als eine von der Verbindung separate Zusammensetzung zur Verabreichung zur gleichen Zeit oder zu einer unterschiedlichen Zeit bereitgestellt ist.

8. Verbindung zur Verwendung nach einem der Ansprüche 1 bis 4, wobei
• 4-{2-[5-Methyl-1-(naphthalen-2-yl)-1H-pyrazol-3-yloxy]ethyl}morpholin oder ein pharmazeutisch akzeptables Salz oder Solvat davon mit einer Tagesdosis von 100 mg bis 600 mg pro Tag während der ersten 5 Tage verabreicht wird, und
• das Taxan mit einer Tagesdosis von zumindest ≥ 60 mg/m² an Tag 1 des Zyklus verabreicht wird.

9. Verbindung zur Verwendung nach einem der Ansprüche 1 bis 5, wobei
• 4-{2-[5-Methyl-1-(naphthalen-2-yl)-1H-pyrazol-3-yloxy]ethyl}morpholin oder ein pharmazeutisch akzeptables Salz, Isomer oder Solvat davon mit einer Tagesdosis von 200 mg bis 400 mg pro Tag während der ersten 5 Tage verabreicht wird, und
• das Taxan mit einer Tagesdosis von zumindest ≥ 80 mg/m² an Tag 1 des Zyklus verabreicht wird.

10. Verbindung zur Verwendung nach einem der Ansprüche 1 bis 5, wobei
• 4-{2-[5-Methyl-1-(naphthalen-2-yl)-1H-pyrazol-3-yloxy]ethyl}morpholin oder ein pharmazeutisch akzeptables Salz, Isomer oder Solvat davon mit einer Tagesdosis von 400 mg pro Tag während der ersten 5 Tage verabreicht wird, und
• das Taxan mit einer Tagesdosis von zumindest 85 mg/m² an Tag 1 des Zyklus verabreicht wird.

## Revendications

1. Composé 4-{2-[5-Méthyl-1-(naphtalén-2-yl)-1H-pyrazol-3-yloxy]éthyl}morpholine ou un sel ou solvate pharmaceutiquement acceptable de celui-ci destiné à être utilisé dans une méthode thérapeutique pour augmenter l'efficacité d'un médicament chimiothérapeutique taxane en réduisant la prolifération des cellules cancéreuses.

2. Le composé à utiliser selon la revendication 1, dans lequel le composé est le chlorhydrate de 4-{2-[5-Méthyl-1-(naphtalén-2-yl)-1H-pyrazol-3-yloxy]éthyl}morpholine ou un solvate de celui-ci.

3. Le composé à utiliser selon l'une quelconque des revendications 1 ou 2, dans lequel le taxane est le Paclitaxel.

4. Le composé à utiliser selon l'une quelconque des revendications 1 à 3, dans lequel le composé est administré en une dose journalière allant de 100 mg à 600 mg par jour.

5. Le composé à utiliser selon la revendication 4, dans lequel le composé est administré en une dose journalière allant de 200 mg à 400 mg par jour.

6. Le composé à utiliser selon l'une quelconque des revendications 1 à 5, dans lequel le taxane fait partie de la même composition que le composé.

7. Le composé à utiliser selon l'une quelconque des revendications 1 à 5, dans lequel le taxane est fourni sous la forme d'une composition séparée du composé pour une administration en même temps ou à un moment différent.

8. Le composé à utiliser selon l'une quelconque des revendications 1 à 4, dans lequel :
• la 4-{2-[5-méthyl-1-(naphtalén-2-yl)-1H-pyrazol-3-yloxy]éthyl}morpholine ou un sel ou solvate pharmaceutiquement acceptable de celle-ci est administrée à une dose journalière allant de 100 mg à 600 mg par jour pendant les 5 premiers jours, et
• le taxane est administré à une dose journalière au moins ≥60 mg/m² le premier jour du cycle.

9. Le composé à utiliser selon l'une quelconque des revendications 1 à 5, dans lequel :
• la 4-{2-[5-méthyl-1-(naphtalén-2-yl)-1H-pyrazol-3-yloxy]éthyl}morpholine ou un sel, un isomère ou un solvate pharmaceutiquement acceptable de celle-ci est administrée à une dose journalière allant de 200 mg à 400 mg par jour pendant les 5 premiers jours, et
• le taxane est administré à une dose journalière de ≥80 mg/m² le premier jour du cycle.

10. Le composé à utiliser selon l'une quelconque des revendications 1 à 5, dans lequel :
• la 4-{2-[5-méthyl-1-(naphtalén-2-yl)-1H-pyrazol-3-yloxy]éthyl}morpholine ou un sel, un isomère ou un solvate pharmaceutiquement acceptable de celle-ci est administrée à une dose journalière de 400 mg par jour pendant les 5 premiers jours, et
• le taxane est administré à une dose journalière à partir de 85 mg/m² le premier jour du cycle.
